# EUROPEAN PATENT SPECIFICATION

(11) **EP 2 155 257 B1**
(45) Date of publication and mention of the grant of the patent: **05.10.2016**
(21) Application number: 08743275.3
(22) Date of filing: 25.04.2008
(51) Int. Cl.: A61K 47/48, C07F 9/6512, A61P 31/20

(54) **METHODS OF REDUCING NEPHROTOXICITY IN SUBJECTS ADMINISTERED WITH NUCLEOSIDE**
VERFAHREN ZUR VERRINGERUNG VON NEPHROTOXIZITÄT BEI PATIENTEN, DENEN EIN NUCLEOSID VERABREICHT WURDE
PROCÉDÉS DE RÉDUCTION DE LA NÉPHROTOXICITÉ CHEZ DES SUJETS AUXQUELS IL A ÉTÉ ADMINISTRÉ UN NUCLÉOSIDE

(30) Priority: 27.04.2007 US 914532 P
(43) Date of publication of application: 24.02.2010
(73) Proprietor: Chimerix, Inc., Durham, NC 27713 (US)
(72) Inventor: PAINTER, George R., Chapel Hill North Carolina 27514 (US)
(74) Representative: Cooley (UK) LLP
(86) International application number: PCT/US2008/005322
(87) International publication number: WO 2008/133966

(56) References cited:
- WO-A-2006/110655
- US-A1- 2004 019 232
- BIDANSET D. J. ET AL.: "Oral activity of ether lipid ester prodrugs of cidofovir against experimental human cytomegalovirus infection" THE JOURNAL OF INFECTIOUS DISEASES, vol. 190, no. 3, 1 August 2004 (2004-08-01), pages 499-503, XP002485780
- PAINTER G R ET AL: "Design and development of oral drugs for the prophylaxis and treatment of smallpox infection" TRENDS IN BIOTECHNOLOGY, ELSEVIER PUBLICATIONS, CAMBRIDGE, GB, vol. 22, no. 8, 1 August 2004 (2004-08-01), pages 423-427, XP004524159 ISSN: 0167-7799
- BIRON ET AL: "Antiviral drugs for cytomegalovirus diseases" ANTIVIRAL RESEARCH, ELSEVIER SCIENCE BV., AMSTERDAM, NL, vol. 71, no. 2-3, 1 September 2006 (2006-09-01), pages 154-163, XP005605076 ISSN: 0166-3542
- DAL POZZO ET AL: "In vitro evaluation of the anti-orf virus activity of alkoxyalkyl esters of CDV, cCDV and (S)-HPMPA" ANTIVIRAL RESEARCH, ELSEVIER SCIENCE BV., AMSTERDAM, NL, vol. 75, no. 1, 17 April 2007 (2007-04-17), pages 52-57, XP022031604 ISSN: 0166-3542
- CIESLA S.L. ET AL.: "Esterification of cidofovir with alkoxyalkanols increases oral bioavailability and diminishes drug accumulation in kidney" ANTIVIRAL RESEARCH, vol. 59, no. 3, August 2003 (2003-08), pages 163-171, XP002485781
- JOSEPHSON M. A. ET AL.: "Polyomavirus-associated nephropathy : Update on antiviral strategies" TRANSPLANT INFECTIOUS DISEASE, vol. 8, no. 2, June 2006 (2006-06), pages 95-101, XP002485782
- RANDHAWA P. ET AL.: "Ether lipid ester derivatives of cidofovir inhibit polyomavirus BK replication in vitro" ANTIMICROBIAL AGENTS AND CHEMOTHERAPY, vol. 50, no. 4, April 2006 (2006-04), pages 1564-1566, XP002485899
- DE CLERCQ ET AL: "The acyclic nucleoside phosphonates from inception to clinical use: Historical perspective" ANTIVIRAL RESEARCH, ELSEVIER SCIENCE BV., AMSTERDAM, NL, vol. 75, no. 1, 17 April 2007 (2007-04-17), pages 1-13, XP022031598 ISSN: 0166-3542
- KERN ET AL: "Pivotal role of animal models in the development of new therapies for cytomegalovirus infections" ANTIVIRAL RESEARCH, ELSEVIER SCIENCE BV., AMSTERDAM, NL, vol. 71, no. 2-3, 1 September 2006 (2006-09-01), pages 164-171, XP005605077 ISSN: 0166-3542
- PARKER ET AL: "Efficacy of therapeutic intervention with an oral ether-lipid analogue of cidofovir (CMX001) in a lethal mousepox model" ANTIVIRAL RESEARCH, ELSEVIER SCIENCE BV., AMSTERDAM, NL, vol. 77, no. 1, 1 January 2008 (2008-01-01), pages 39-49, XP022405846 ISSN: 0166-3542
- PAINTER G. R. ET AL.: "Evaluation of hexadecyloxypropyl-9-R-[2-(phosphonomethox y)propyl]-adenine, CMX157, as a potential treatment for human immunodeficiency virus type 1 and hepatitis B virus infections" ANTIMICROBIAL AGENTS AND CHEMOTHERAPY, vol. 51, no. 10, October 2007 (2007-10), pages 3505-3509, XP002485783
- JACOBSON M. A.: "Treatment of cytomegalovirus retinis in patients with the acquired immunodeficiency syndrome" DRUG THERAPY, vol. 337, no. 2, 1997, pages 105-114, XP002485817
- HOSTETLER K Y ET AL: "Alkoxyalkyl esters of (S)-9-[3-hydroxy-2-(phosphonomethoxy)propy l]ad enine are potent inhibitors of the replication of wild-type and drug-resistant human immunodeficiency virus type 1 in vitro", ANTIMICROBIAL AGENTS AND CHEMOTHERAPY, AMERICAN SOCIETY FOR MICROBIOLOGY, WASHINGTON, DC, US LNKD- DOI:10.1128/AAC.01223-05, [Online] vol. 50, no. 8, 1 August 2006 (2006-08-01) , pages 2857-2859, XP008128379, ISSN: 0066-4804 Retrieved from the Internet: URL:http://aac.asm.org/ ORD - 2006-08-00>
- HOSTETLER K.Y. ET AL.: 'In vitro and in vivo evaluation of hexadecyloxypropyl-9-R-[2-(phosphonomethoxy )propyl]adenine as potential treatment of HIV-1 infection' GLOBAL ANTIVIRAL JOURNAL vol. 2, no. 2, 2006, pages 96 - 97, XP007915532
- HELD T K ET AL: "Treatment of BK virus-associated hemorrhagic cystitis and simultaneous CMV reactivation with cidofovir", BONE MARROW TRANSPLANTATION, NATURE PUBLISHING GROUP, GB, [Online] vol. 26, no. 3, 1 January 2000 (2000-01-01), pages 347-350, XP008128380, ISSN: 0268-3369 Retrieved from the Internet: URL:http://www.nature.com/bmt/ ORD - 2000-00-00>

## Description

### Field of the Invention

The present invention concerns a nucleoside phosphonate compound for use in the treatment of adenovirus in an immunodeficient subject.

### Background of the Invention

Cidofovir (VISTIDE®) is a nucleoside analog approved by the US FDA for the treatment of CMV retinitis in patients with AIDS. It is active against all dsDNA viruses that cause human disease. Cidofovir has the structure:

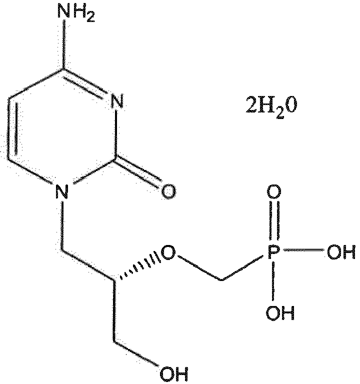

Cidofovir requires intravenous infusion and is dose-limited by its nephrotoxicity. Cases of acute renal failure resulting in dialysis and/or contributing to death have occurred with as few as one or two doses of VISTIDE® Cidofovir. *See, e.g.,* Gilead Letter, Important Drug Warning (Sept. 1996) (available from the US FDA). Hence, prehydration with normal saline and probenecid co-administration are required with Cidofovir therapy. *See, e.g.,* S. Lacy, Toxicological Sciences 44, 97-106 (1998).

US Patent Nos. 6,716,825; 7,034,014; 7,094,772; and 7,098,197, to Hostetler et al. describe lipid conjugates of phosphonate compounds, including cidofovir, for the treatment of disease. Bidanset D. et al, The Journal of Infectious Diseases, 2004; 190:499-503 discusses the oral activity of ether lipid ester prodrugs of cidofovir against experimental human cytomegalovirus infection.

### SUMMARY OF THE INVENTION

The present invention provides a compound: 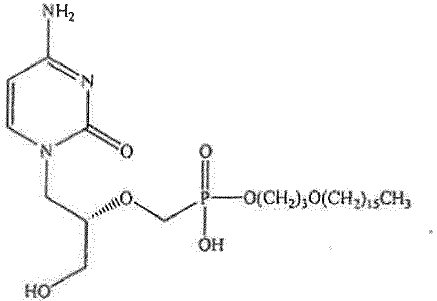 or a pharmaceutically acceptable salt thereof, for use in the therapeutic and/or prophylactic treatment of adenovirus in an immunodeficient subject, wherein said immunodeficient subject is a human. The immunodeficient subject may have primary or acquired immunodeficiency.

In one embodiment of the invention, the immunodeficient subject has acquired immunodeficiency as a result of immunosuppressive therapy. Cyclosporine for example is an immunosuppressant drug widely used in post-allogeneic organ transplant to reduce the activity of the patient's immune system and so the risk of organ rejection. In one embodiment of the invention therefore, the immunodeficient subject is a transplant patient. The immunodeficient subject may be a renal transplant patient, a hepatic transplant patient or a bone marrow transplant patient. In an alternative embodiment of the invention, the subject is suffering from chronic fatigue syndrome.

In one embodiment of the invention, the viral infection to be treated is resistant to treatment with an unconjugated acyclic nucleoside phosphonate, e.g., cidofovir, cyclic cidofovir, tenofovir, and adefovir, *etc.* Alternatively or additionally, an unconjugated acyclic nucleoside phosphonate exhibits toxic side effects in said immunodeficient subject.

In another embodiment of the invention, the immunodeficient subject is infected with two or more viruses, at least one of which is preferably a dsDNA virus, and the viruses exhibit synergistic action.

Preferably the conjugate compound is used to treat a dsDNA viral infection in an immunodeficient subject wherein said subject is resistant to valganciclovir hydrochloride (or ganciclovir) or wherein said subject exhibits side effects to valganciclovir hydrochloride (or ganciclovir).

The patient may be a bone marrow stem cell transplant patient , especially where there is a risk (real or perceived) for bone marrow toxicity from ganciclovir in the patient.

Preferably the conjugate compound of the invention is administered orally, preferably at a dosage of less than 5 mg/Kg, more preferably at a dosage of less than 1 mg/Kg. More preferably said conjugate compound is administered to said subject at a dosage of 10 or 20, up to 200 or 300 or up to 5000 ug/Kg. The lipid conjugates of the invention can be administered daily, every other day, once a week or once every 2 weeks.

### Brief Description of the Drawings

**Figure 1** shows plasma concentration curves of CMX001 following a single dose administration; and
**Figure 2****,** shows plasma concentration curves of Cidofovir following a single dose of CMX001.

### Detailed Description of the Invention

As noted above, the present invention provides a compound: 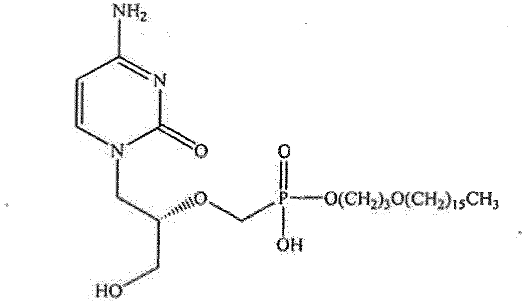 or a pharmaceutically acceptable salt thereof, for use in the therapeutic and/or prophylactic treatment of adenovirus in an immunodeficient subject, wherein said immunodeficient subject is human. In some embodiments, the conjugate compound is administered to said subject at a dosage of less than 1mg/Kg; in some embodiments the conjugate compound is administered to said subject at a dosage of 10 or 20 up to 200 or 300 µg/Kg.

In some embodiments, the present invention is particularly useful in treating subjects afflicted with at least two different viruses or two different dsDNA viruses, which synergistically activate one another. *See, e.g.,* LT Feldman et al., PNAS, Aug 15, 1982, 4952-4956; B. Bielora et al., Bone Marrow Transplant, 2001 Sep; 28(6): 613-614.

The present invention is explained in greater detail below.

### A. Definitions.

"Alkyl" as used herein refers to a monovalent straight or branched chain or cyclic radical of from one to twenty-four carbon atoms, including methyl, ethyl, n-propyl, isopropyl, n-butyl, isobutyl, tert-butyl, n-hexyl, and the like.

"Substituted alkyl" as used herein comprises alkyl groups further bearing one or more substituents selected from hydroxy, alkoxy (of a lower alkyl group), mercapto (of a lower alkyl group), cycloalkyl, substituted cycloalkyl, heterocyclic, substituted heterocyclic, aryl, substituted aryl, heteroaryl, substituted heteroaryl, aryloxy, substituted aryloxy, halogen, trifluoromethyl, cyano, nitro, nitrone, amino, amido, -C(O)H, acyl, oxyacyl, carboxyl, carbamate, sulfonyl, sulfonamide, sulfuryl, and the like.

"Alkenyl" as used herein refers to straight or branched chain hydrocarbyl groups having one or more carbon-carbon double bonds, and having in the range of about 2 up to 24 carbon atoms, and "substituted alkenyl" refers to alkenyl groups further bearing one or more substituents as set forth above.

"Aryl" as used herein refers to aromatic groups having in the range of 6 up to 14 carbon atoms and "substituted aryl" refers to aryl groups further bearing one or more substituents as set forth above.

"Heteroaryl" as used herein refers to aromatic groups containing one or more heteroatoms (e.g., N, O, S, or the like) as part of the ring structure, and having in the range of 3 up to 14 carbon atoms and "substituted heteroaryl" refers to heteroaryl groups further bearing one or more substituents as set forth above.

"Bond" or "valence bond" as used herein refers to a linkage between atoms consisting of an electron pair.

"Pharmaceutically acceptable salts" as used herein refers to both acid and base addition salts.

"Prodrug" as used herein refers to derivatives of pharmaceutically active compounds that have chemically or metabolically cleavable groups and become the pharmaceutically active compound by solvolysis or under in vivo physiological conditions.

"Parenteral" as used herein refers to subcutaneous, intravenous, intra-arterial, intramuscular or intravitreal injection, or infusion techniques.

"Topically" as used herein encompasses administration rectally and by inhalation spray, as well as the more common routes of the skin and mucous membranes of the mouth and nose and in toothpaste.

"Eeffective amount" as used herein as applied to the phosphonate prodrugs of the invention is an amount that will prevent or reverse the disorders noted above. Particularly with respect to disorders associated with bone metabolism, an effective amount is an amount that will prevent, attenuate, or reverse abnormal or excessive bone resorption or the bone resorption that occurs in the aged, particularly post-menopausal females or prevent or oppose bone metastasis and visceral metastasis in breast cancer.

"Immunodeficiency" (or " immune deficiency") as used herein refers to a state in which the ability of the immune system to fight infectious disease is compromised or entirely absent. A person who has an immunodeficiency of any kind is said to be immunocompromised. An immunocompromised person may be particularly vulnerable to opportunistic infections, in addition to normal infections.

"Treatment" as used herein includes any procedure with a purpose to prevent, pre-empt, treat or cure a disease. Prophylactic treatment may include either *primary* prophylaxis (to prevent the development of a disease) and/or *secondary* prophylaxis (whereby the disease has already developed and the patient is protected against worsening of this process).

### B. Compounds.

Further compounds, compositions and formulations are described herein and in US Patent No. 6,716,825; 7,034,014; 7,094,772; and 7,098,197.

Phosphonate compounds may have the structure: 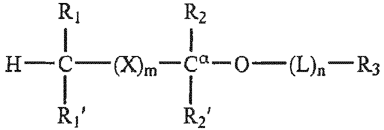 wherein:
R₁ and R₁' are independently -H, optionally substituted -O(C₁-C₂₄)alkyl, -O(C₁-C₂₄)alkenyl, -O(C₁-C₂₄)acyl, -S(C₁-C₂₄)alkyl, -S(C₁-C₂₄)alkenyl, or -S(C₁-C₂₄)acyl, wherein at least one of R₁ and R₁' are not -H, and wherein said alkenyl or acyl moieties optionally have 1 to 6 double bonds,
R₂ and R₂' are independently -H, optionally substituted -O(C₁-C₇)alkyl, -O(C₁-C₇)alkenyl, -S(C₁-C₇)alkyl, -S(C₁-C₇)alkenyl, -O(C₁-C₇)acyl, -S(C₁-C₇)acyl,-N(C₁-C₇)acyl, -NH(C₁-C₇)alkyl, -N((C₁-C₇)alkyl)₂, oxo, halogen, -NH₂, -OH, or-SH;
R₃ is a pharmaceutically active phosphonate, bisphosphonate or a phosphonate derivative of a pharmacologically active compound, linked to a functional group on optional linker L or to an available oxygen atom on C_{α};
X, when present, is: 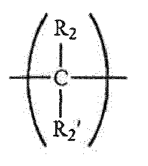
L is a valence bond or a bifunctional linking molecule of the formula -J-(CR₂)ₜ-G-, wherein t is an integer from 1 to 24, J and G are independently -O-, -S-, -C(O)O-, or -NH-, and R is -H, substituted or unsubstituted alkyl, or alkenyl;
m is an integer from 0 to 6; and
n is 0 or 1.
Optimally, m=0, 1 or 2, R₂ and R₂' are preferably
H, and the prodrugs are then ethanediol, propanediol or butanediol derivatives of a therapeutic phosphonate. A preferred ethanediol phosphonate species has the structure: 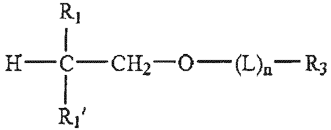 wherein R₁, R₁', R₃, L, and n are as defined above.

One propanediol species has the structure: 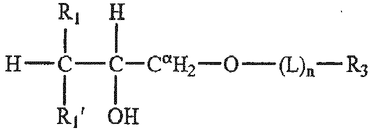 wherein m=1 and R₁, R₁', R₃, L and n are as defined above in the general formula. A glycerol species has the structure: 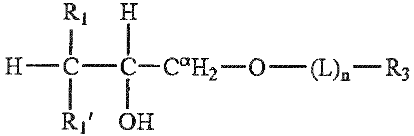
wherein m=1, R₂=H, R₂'=OH, and R₂ and R₂' on C^{α} are both -H. Glycerol is an optically active molecule. Using the stereospecific numbering convention for glycerol, the sn-3 position is the position which is phosphorylated by glycerol kinase. In compounds
having a glycerol residue, the -(L)ₙ-₃ moiety may be joined at either the sn-3 or sn-1 position of glycerol.

In all species of the pharmacologically active agents, R₁ is preferably an alkoxy group having the formula -O-(CH₂)ₜ-CH₃, wherein t is 0-24. More preferably t is 11-19. Most preferably t is 15 or 17.

Some examples of antiviral phosphonates derived by substituting -CH₂-PO₃H₂ for the 5'-hydroxyl are: AZT phosphonate, d4T phosphonate, ddC phosphonate, Adefovir, ganciclovir phosphonate, acyclovir phosphonate, ganciclovir cycloic phosphonate, and 3'-thia-2',3'-dideoxycytidine-5'-phosphonic acid.

Other examples of antiviral nucleotide phosphonates
are derived similarly from antiviral nucleosides including ddA, ddI, ddG, L-FMAU, DXG, DAPD, L-dA, L-dI, L-(d)T, L-dC, L-dG, FTC, penciclovir, and the like.

Additionally, antiviral phosphonates such as cidofovir, cyclic cidofovir, adefovir, tenofovir, and the like, may be used as an R₃ group.

Certain compounds possess one or more chiral centers, e.g. in the sugar moieties, and may thus exist in optically active forms. Likewise, when the compounds contain an alkenyl group or an unsaturated alkyl or acyl moiety there exists the possibility of cis- and trans-isomeric forms of the compounds. Additional asymmetric carbon atoms can be present in a substituent group such as an alkyl group. The R- and S-isomers and mixtures thereof, including racemic mixtures as well as mixtures of cis- and trans-isomers are disclosed.

If a particular stereoisomer is desired, it can be prepared by methods well known in the art by using stereospecific reactions with starting materials that contain the asymmetric centers and are already resolved or, alternatively, by methods that lead to mixtures of the stereoisomers and resolution by known methods.

Nucleosides useful for treating viral infections may also be converted to their corresponding 5 '-phosphonates for use as an R₃ group. Such phosphonate analogs typically contain either a phosphonate (-PO₃H₂) or a methylene phosphonate (-CH₂-PO₃H₂) group substituted for the 5'-hydroxyl of an antiviral nucleoside. Some examples of antiviral phosphonates derived by substituting -PO₃H₂ for the 5'-hydroxyl are:
Many phosphonate compounds exist that can be derivatized
   to improve their pharmacologic activity, or to increase their oral absorption, such as, for example, the compounds disclosed in the following patents:
   U.S. Pat. No. 5,043,437 (Phosphonates of azidodideoxynucleosides), U.S. Pat. No. 5,047,533 (Acyclic purine phosphonate nucleotide analogs), U.S. Pat. No. 5,142,051 (N-Phosphonylmethoxyalkyl derivatives of pyrimidine and purine bases), U.S. Pat. No. 5,247,085 (Antiviral purine compounds), U.S. Pat. No. 5,395,826 (Guanidinealky 1-1,1-bisphosphonic acid derivatives), U.S. Pat. No. 5,656,745 (Nucleotide analogs), U.S. Pat. No. 5,672,697 (Nucleoside-5'-methylene phosphonates), U.S. Pat. No. 5,717,095 (Nucleotide analogs), U.S. Pat. No. 5,760,013 (Thymidylate analogs), U.S. Pat. No. 5,798,340 (Nucleotide analogs), U.S. Pat. No. 5,840,716 (Phosphonate nucleotide compounds), U.S. Pat. No. 5,856,314 (Thio-substituted, nitrogen-containing, heterocyclic phosphonate compounds), U.S. Pat. No. 5,885,973 (olpadronate), U.S. Pat. No. 5,886,179 (Nucleotide analogs), U.S. Pat. No. 5,877,166 (Enantiomericaily pure 2-aminopurine phosphonate nucleotide analogs), U.S. Pat. No. 5,922,695 (Antiviral phosphonomethoxy nucleotide analogs), U.S. Pat. No. 5,922,696 (Ethylenic and allenic phosphonate derivatives of purines), U.S. Pat. No. 5,977,089 (Antiviral phosphonomethoxy nucleotide analogs), U.S. Pat. No. 6,043,230 (Antiviral phosphonomethoxy nucleotide analogs), U.S. Pat. No. 6,069,249 (Antiviral phosphonomethoxy nucleotide analogs); Belgium Patent No. 672205 (Clodronate); European Patent No. 753523 (Amino-substituted bisphosphonic acids); European Patent Application 186405 (geminal diphosphonates); and the like.

Phosphonate analogs, comprising therapeutically effective phosphonates (or phosphonate derivatives of therapeutically effective compounds) covalently linked by a hydroxyl group to a 1-O-alkyglycerol, 3-O-alkylglycerol, 1-S-alkylthioglycerol, or alkoxy-alkanol, may be absorbed more efficiently in the gastrointestinal tract than are the parent compounds. An orally administered dose of the analog is taken up intact from the gastrointestinal tract of a mammal and the active drug is released in vivo by the action of endogenous enzymes. Phosphonate analogs may also have a higher degree of bioactivity than the corresponding underivatized compounds.

The compounds of the present invention are an improvement over alkylglycerol phosphate prodrugs described in the prior art because the phosphonate-containing moiety is linked directly to the alkyl-glycerol or the alkoxy-alkanol moiety and because the presence of the phosphonate bond prevents enzymatic conversion to the free drug. Other linkers between these groups can be present in the improved analogs. For example, bifunctional linkers having the formula -O-(CH₂)ₙ-C(O)O- wherein n is 1 to 24, can connect the phosphonate to the hydroxyl group of the alkoxy-alkanol or alkylglycerol moiety.

The foregoing allows the phosphonate of the invention to achieve a higher degree of oral absorption. Furthermore, cellular enzymes, but not plasma or digestive tract enzymes, will convert the conjugate to a free phosphonate. A further advantage of the alkoxy-alkanol phosphonates is that the tendency of co-administered food to reduce or abolish phosphonate absorption is greatly reduced or eliminated, resulting in higher plasma levels and better compliance by patients.

Compounds (or "prodrugs") can be prepared in a variety of ways, as generally depicted in **Schemes I -VI** of US Patent No. 6,716,825. The general phosphonate esterification methods described below are provided for illustrative purposes. Indeed, several methods have been developed for direct condensation of phosphonic acids with alcohols (*see,* for example, R. C. Larock, Comprehensive Organic Transformations, VCH, New York, 1989, p. 966 and references cited therein). Isolation and purification of the compounds and intermediates described in the examples can be effected, if desired, by any suitable separation or purification procedure such as, for example, filtration, extraction, crystallization, flash column chromatography, thin-layer chromatography, distillation or a combination of these procedures. Specific illustrations of suitable separation and isolation procedures are in the examples below. Other equivalent separation and isolation procedures can of course, also be used.

**Scheme I** of US Patent No. 6,716,825. outlines a synthesis of bisphosphonate prodrugs that contain a primary amino group, such as pamidronate or alendronate. Example 1 therein provides conditions for a synthesis of 1-O-hexadecyloxypropyl-alendronate (HDP-alendronate) or 1-O-hexadecyloxypropyl-pamidronate (HDP-pamidronate). In this process, a mixture oT dimethyl 4-phthalimidobutanoyl phosphonate (lb, prepared as described in U.S. Pat. No. 5,039,819)) and hexadecyloxypropyl methyl phosphite (2) in pyridine solution is treated with triethylamine to yield bisphosphonate tetraester 3b which is purified by silica gel chromatography. Intermediate 2 is obtained by transesterification of diphenyl phosphite as described in Kers, A., Kers, I., Stawinski, J., Sobkowski, M., Kraszewski, A. Synthesis, April 1995, 427 430. Thus, diphenyl phosphite in pyridine solution is first treated with hexadecyloxypropan-1-ol, then with methanol to provide compound 2.

An important aspect of the process is that other long chain alcohols may be used in place of hexadecyloxypropan-1-ol to generate various compounds. Treatment of intermediate 3b with bromotrimethylsilane in acetonitnle cleaves the methyl esters selectively to yield monoester 4b. Treatment of 4b with hydrazine in a mixed solvent system (20% melhanol/80% 1,4-dioxane) results in removal of the phthalimido protecting group as shown. The desired alendronate prodrug is collected by filtration and converted to the triammonium salt by treatment with methanolic ammonia.

**Scheme II** of US Patent No. 6,716,825. illustrates a synthesis of analogs of bisphosphonates lacking a primary amino group, hi this case the process steps are similar to those of Scheme 1 except that protection with a phthalimido group and subsequent deprotection by hydrazinolysis are unnecessary.

Bisphosphonates having 1-amino groups, such as amino-olpadronate, maybe converted to analogs using a slightly modified process shown in **Scheme III** of US Patent No. 6,716,825. Treatment of a mixture of compound 2 and 3-(dimethylamino)propionitrile with dry HCl followed by addition of dimethyl phosphite affords tetraester 3 which, after demethylation with bromotrimethylsilane, yields hexadecyloxypropyl-amino-olpadronate.

**Scheme IV** of US Patent No. 6,716,825 illustrates synthesis of a bisphosphonate analog where the lipid group is attached to a primary amino group of the parent compound rather than as a phosphonate ester.

**Scheme V** of US Patent No. 6,716,825. illustrates a general synthesis of alkylglycerol or alkylpropanediol analogs of cidofovir, cyclic cidofovir, and other phosphonates. Treatment of 2,3-isopropylidene glycerol, 1, with NaH in dimethylformamide followed by reaction with an alkyl methanesulfonate yields the alkyl ether, 2. Removal of the isopropylidene group by treatment with acetic acid followed by reaction with trityl chloride in pyridine yields the intermediate 3. Alkylation of intermediate 3 with an alkyl halide results in compound 4. Removal of the trityl group with 80% aqueous acetic acid affords the O,O-dialkyl glycerol, 5. Bromination of compound 5 followed by reaction with the sodium salt of cyclic cidofovir or other phosphonate-containing nucleotide yields the desired phosphonate adduct, 7. Ring-opening of the cyclic adduct is accomplished by reaction with aqueous sodium hydroxide. The preferred propanediol species may be synthesized by substituting 1-O-alkylpropane-3-ol for compound 5 in Scheme V. The tenofovir and adefovir analogs may be synthesized by substituting these nucleotide phosphonates for cCDV in reaction (f) of Scheme V.

**Scheme VI** of US Patent No. 6,716,825. illustrates a general method for the synthesis of nucleotide phosphonates using 1-O-hexadecyloxypropyl-adefovir as the example. The nucleotide phosphonate (5 mmol) is suspended in dry pyridine and an alkoxyalkanol or alkylglycerol derivative (6 mmol) and 1,3-dicyclohexylcarbodiimde (DCC, 10 mmol) are added. The mixture is heated to reflux and stirred vigorously until the condensation reaction is complete as monitored by thin-layer chromatography. The mixture is then cooled and filtered. The filtrate is concentrated under reduced pressure and the residues adsorbed on silica gel and purified by flash column chromatography (elution with approx. 9:1 dichloromethane/methanol) to yield the corresponding phosphonate monoester.

### C. Compositions.

Compounds of the invention can be administered orally in the form of tablets, capsules, solutions, emulsions or suspensions, inhaled liquid or solid particles, microencapsulated particles, as a spray, through the skin by an appliance such as a transdermal patch, or rectally, for example, in the form of suppositories. The lipophilic prodrug derivatives of the compounds are particularly well suited for transdermal absorption administration and delivery systems and may also be used in toothpaste. Administration can also take place parenterally in the form of injectable solutions.

The compositions may be prepared in conventional forms, for example, capsules, tablets, aerosols, solutions, suspensions, or together with carriers for topical applications. Pharmaceutical formulations containing compounds of this invention can be prepared by conventional techniques, e.g., as described in Remington's Pharmaceutical Sciences, 1985.

The pharmaceutical carrier or diluent employed may be a conventional solid or liquid carrier. Examples of solid carriers are lactose, sucrose, talc, gelatin, agar, pectin, acacia, magnesium stearate, stearic acid, or lower alkyl ethers of cellulose. Examples of liquid carriers are syrup, peanut oil, olive oil, phospholipids, fatty acids, fatty acid amines, polyoxyethylene or water. The carrier or diluent may include any sustained release material known in the art, such as glyceryl monostearate or distearate, alone or mixed with a wax.

If a solid carrier is used for oral administration, the preparation may be tableted or placed in a hard gelatin capsule in powder or pellet form. The amount of solid carrier will vary widely, but will usually be from about 25 mg to about 1 gm. If a liquid carrier is used, the preparation may be in the form of a syrup, emulsion, soft gelatin capsule, or sterile injectable liquid such as an aqueous or non-aqueous liquid suspension or solution.

Tablets are prepared by mixing the active ingredient (that is, one or more compounds of the invention), with pharmaceutically inert, inorganic or organic carrier, diluents, and/or excipients. Examples of such excipients which can be used for tablets are lactose, maize starch or derivatives thereof, talc, stearic acid or salts thereof. Examples of suitable excipients for gelatin capsules are vegetable oils, waxes, fats, semisolid, and liquid polyols. The bisphosphonate prodrugs can also be made in microencapsulated form.

For nasal administration, the preparation may contain a compound of the invention dissolved or suspended in a liquid carrier, in particular, an aqueous carrier, for aerosol application. The carrier may contain solubilizing agents such as propylene glycol, surfactants, absorption enhancers such as lecithin or cyclodextrin, or preservatives.

Pharmaceutical compositions of this invention for parenteral injection comprise pharmaceutically acceptable sterile aqueous or non-aqueous liquids, dispersions, suspensions or emulsions as well as sterile powders for reconstitution into sterile injectable solutions or dispersions just prior to use.

Suitable excipients for the preparation of solutions and syrups are water, polyols, sucrose, invert sugar, glucose, and the like. Suitable excipients for the preparation of injectable solutions are water, alcohols, polyols, glycerol, vegetable oils, and the like.

The pharmaceutical products can additionally contain any of a variety of added components, such as, for example, preservatives, solubilizers, stabilizers, wetting agents, emulsifiers, sweeteners, colorants, flavorings, buffers, coating agents, antioxidants, diluents, and the like.

Optionally, the pharmaceutical compositions may comprise a compound of the invention combined with one or more compounds exhibiting a different activity, for example, an antibiotic or other pharmacologically active material.

### D. Subjects and methods.

A number of rare diseases feature a heightened susceptibility to infections from childhood onward. Many of these disorders are hereditary and are autosomal recessive or X-linked. There are over 80 recognised primary immunodeficiency syndromes; they are generally grouped by the part of the immune system that is malfunctioning, such as lymphocytes or granulocytes. The treatment of primary immunodeficiencies depends on the nature of the defect, and may involve antibody infusions, long-term antibiotics and (in certain cases) stem cell transplantation.

Immune deficiency may also be the result of particular external processes or diseases; the resultant state is called "secondary" or "acquired" immunodeficiency. Common causes for secondary immunodeficiency are malnutrition, aging and particular medications (e.g. chemotherapy, disease-modifying antirheumatic drugs, immunosuppressive drugs after organ transplants, glucocorticoids).

Many specific diseases directly or indirectly impair the immune system. This include many types of cancer, particularly those of the bone marrow and blood cells (leukemia, lymphoma, multiple myeloma), and certain chronic infections. Immunodeficiency is also the hallmark of acquired immunodeficiency syndrome (AIDS), caused by the human immunodeficiency virus (HIV). HIV directly attacks the immune system.

Human cytomegalovirus (HCMV) is a member of the herpes virus family. These dsDNA viruses typically cause mild or subclincal disease, but can cause severe systemic or localised disease in immmunocomprised individuals. All herpes viruses share a characteristic ability to remain latent within the body over long periods. Although primary CMV infection in an immunocompromised patient can cause serious disease, the more common problem is the reactivation of the latent virus.

Immunocompromised patients include organ transplant recipients, patients undergoing hemodialysis, patients with cancer, patients receiving immunosuppressive drugs, and HIV-infected patients. Exposure of immunosuppressed patients to outside sources of CMV should be minimized. Whenever possible, patients without CMV infection should be given organs and/or blood products that are free of the virus.

Patients without CMV infection who are given organ transplants from CMV-infected donors should be given prophylactic treatment with valganciclovir (ideally) or ganciclovir and require regular serological monitoring to detect a rising CMV titre, which should be treated early to prevent a potentially life-threatening infection becoming established.

However, despite prophylaxis, often continued for 100 days, disease prevalence at six months is estimated to be from 12 to 22 percent. Safe and efficacious prophylaxis of CMV infection in transplant patients is not possible with current treatments.

CMX001 has a decided advantage over current treatments for prophylaxis of CMV infection in transplant recipients and cancer patients receiving myelosuppressive chemotherapy or radiation therapy, based on the demonstrated lack of nephrotoxicity at potentially effective concentrations. Large patient populations with needs that could previously not be met with existing therapies can now be treated. The surprisingly low levels of nephrotoxicity associated with the compounds of the present invention means that the application of these compounds in treatment of immunocompromised individuals is now possible.

Furthermore, although anti-viral therapies have advanced substantially in recent years, resistance to current agents and significant drug side effects remain an issue for many patients. The conjugate compounds of the present invention demonstrate high oral bioavailability at lower doses than conventional drugs and this has important implications for disease resistance.

Prodrugs can be administered orally, parenterally, topically, rectally, and through other routes, with appropriate dosage units, as desired.

With respect to disorders associated with viral infections, the "effective amount" is determined with reference to the recommended dosages of the antiviral compound. The selected dosage will vary depending on the activity of the selected compound, the route of administration, the severity of the condition being treated, and the condition and prior medical history of the patient being treated. However, it is within the skill of the art to start doses of the compound(s) at levels lower than required to achieve the desired therapeutic effect and to gradually increase the dosage until the desired effect is achieved. If desired, the effective daily dose may be divided into multiple doses for purposes of administration, for example, two to four doses per day. It will be understood, however, that the specific dose level for any particular patient will depend on a variety of factors, including the body weight, general health, diet, time, and route of administration and combination with other drugs, and the severity of the disease being treated.

Generally, the compounds of the present invention are dispensed in unit dosage form comprising 1% to 100% of active ingredient. The range of therapeutic dosage is from about 0.01 to about 1,000 mg/kg/day with from about 0.10 mg/kg/day to 100 mg/kg/day being preferred, when administered to patients, e.g., humans, as a drug. Actual dosage levels of active ingredients in the pharmaceutical compositions of this invention may be varied so as to administer an amount of the active compound(s) that is effective to achieve the desired therapeutic response for a particular patient.

The present invention is explained in greater detail in the following Examples.

### EXAMPLE 1

### Preclinical Studies of CMX001

As summarized in **Tables 1-2** below, pre-clinical studies of CMX001 indicate that it is essentially completely protective against lethal Orthopoxivirus infections in mice and rabbits. The effective dose in these animal models ranges from 1-2 mg/kg daily for 5 days in low titer inoculums, while late stage requires 20-30 mg/kg as a single dose.

| **Table 1: CMX001 has Enhanced In Vitro Potency Against dsDNA Viruses.** | | | | |
|---|---|---|---|---|
| **Virus** | **Cell Line** | **Cidofovir EC50 (µM)** | **CMX001 EC50 (µM)** | **Enhanced Activity** |
| Variola major | Vero 76 | 27.3 | 0.1 | 271 |
| Vaccinia Virus | HFF | 46 | 0.8 | 57 |
| HCMV(AD169) | MRC-5 | 0.38 | 0.0009 | 422 |
| BK Virus | WI-38 | 115.1 | 0.13 | 885 |
| HSV-1 | MRC-5 | 15 | 0.06 | 250 |
| HHV-6 | HSB-2 | 0.2 | 0.004 | 50 |
| Adenovirus | HFF | 1.3 | 0.02 | 65 |
| HPV 18 | HeLa | 516 | 0.42 | 1229 |
| HPV 11 | A431 | 716 | 17 | 42 |
| EBV | Dardi | >170 | 0.04 | >4250 |

| **Table 2: CMX001 is protective against lethal orthopoxivirus infections in mice and rabbits.** | | | | |
|---|---|---|---|---|
| | | | **Viral Inoculum (PFU)** | **100% Protective Dose of CMX001*** |
| **Mice Infected with Ectromelia** | | | 1.2 | 1 mg/kg/day |
| | | | 27 | 4 mg/kg/day |
| | | | 270 | 4 mg/kg/day |
| | | | 9200 | 8 mg/kg/day |
| **Rabbits Infected with Rabbitpox** | | | 100 | 2 mg/kg/day |
| | | | 500 | 10 mg/kg/day |
| | | | 1000 | 20 mg/kg/day |

| | | | | |
|---|---|---|---|---|
| *Dose was orally administered for five consecutive days | | | | |

In addition, over twenty-one toxicology studies have been conducted in mice, rats, rabbits and monkeys with CMX001 being delivered by the oral route. In none of these studies (as opposed to the delivery of efficacious doses of cidofovir by i.v.), has there been any indication of nephrotoxicity (*see, e.g.,* Example 2 below).

### EXAMPLE 2

### Clinical Studies

An initial study was conducted to evaluate the safety and pharmacokinetics of CMX001 in healthy volunteers. The study consisted of a single dose arm (SD) and a multiple dose arm (MD). In the single dose arm 7 cohorts of 6 subjects were treated (4 subjects received active drug and 2 placebo). Enrollment was staggered as 2 subjects (one active, one placebo) followed by 4 subjects (Groups A and B). The estimated single doses for the two highest doses treated for a 75 kg subject were 40 mg (0.6mg/kg cohort 6) and 70 mg (1mg/kg cohort 7). In the multiple dose arm, cohort 6MD received 0.1 mg/kg on Day 0, 6 and 12; Cohort 7MD received 0.2 mg/kg on Day 0, 6 and 12. Levels of cidofovir, CMX001 and CMX064 (major metabolite) were measured in blood and urine of subjects during the course of the study. Gastrointestinal (GI) monitoring of the subjects included (a) monitoring for clinical signs of GI adverse events, (b) monitoring for clinical symptoms using a visual Analog Scale, (c) monitoring for appetite loss/anorexia, nausea, vomiting, diarrhea, constipation and intestinal gas/bloating, (d) laboratory tests for fecal occult blood; serum electrolytes, urine specific gravity, BUN/creatinine ratio; serum albumin, and lipids, and (e) diagnostic studies (the Wireless capsule endoscopy (PillCam®, Given Imaging)).

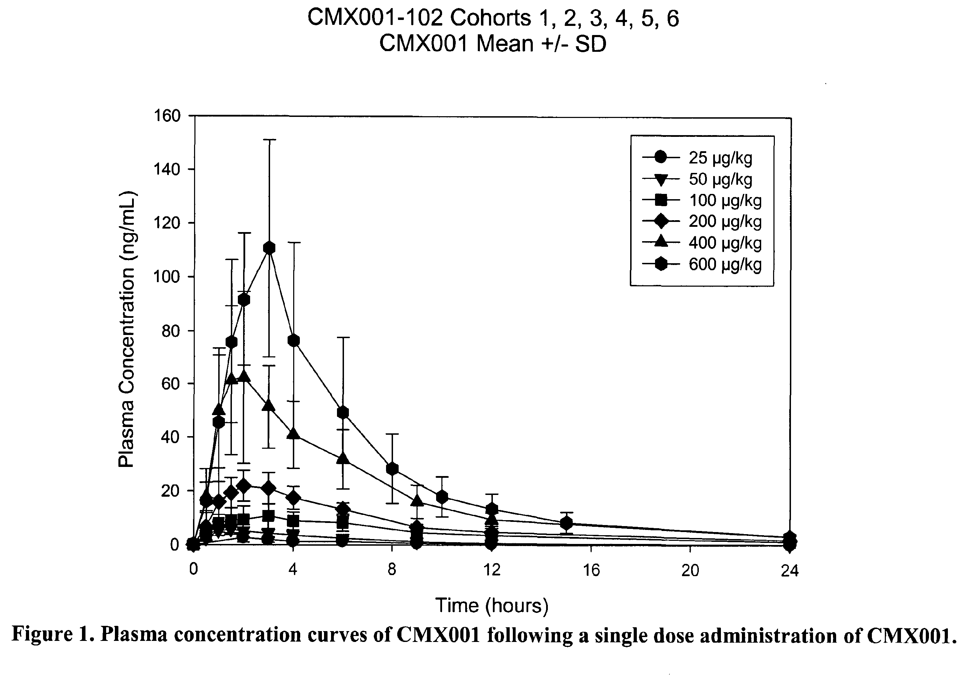

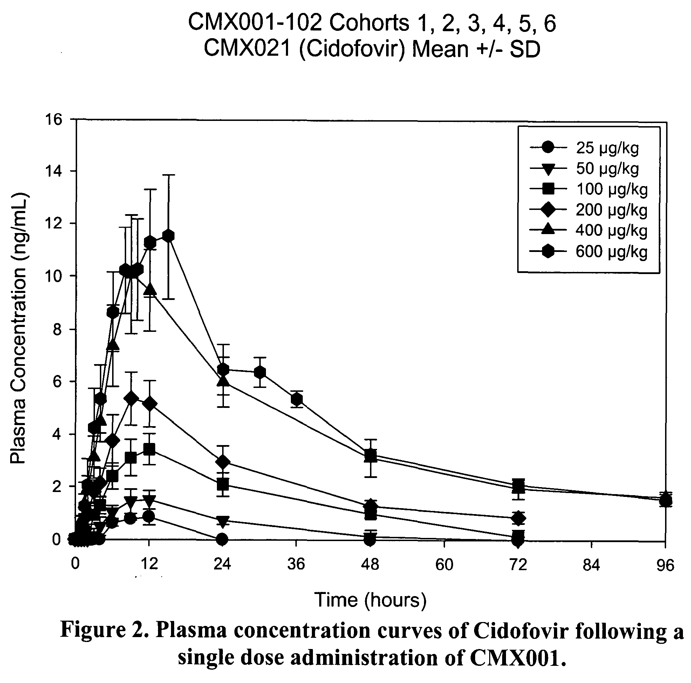

Upon the completion of the study of cohort 6 (600µg/kg) (while still blinded) it was observed as follows:
No post-dose clinically significant gastrointestinal capsule endoscopy findings attributable to drug.
No drug associated clinically significant changes to clinical laboratory values, including those indicative of kidney dysfunction.
No serious adverse events (SAEs), no significant adverse events (AEs) (i.e. ≥ Grade 2), no AEs directly attributable to drug.
Plasma concentration curves of CMX001 following a single dose administration are shown in **Figure 1****,** and plasma concentration curves of Cidofovir following a single dose of CMX001 are shown in **Figure 2****.**

**Table 3** illustrates the PK comparison of CMX 001 with CMX 021 and CMX 064 for mouse, rabbit and human.

**TABLE 3**

| **Species** | **Dose (mg/kg)** | **CMX001** | | **CMX021** | | **CMX064** | |
|---|---|---|---|---|---|---|---|
| | | **Cmax (ng/mL)** | **AUCO→ (ng*h/mL)** | **Cmax (ng/mL)** | **AUCO→ (ng*h/mL)** | **Cmax (ng/mL)** | **AUCO→ (ng*h/mL)** |
| **Mouse-Rabbit** | 2 | 7.9-18.0 | 83.14-102.4 | BQL-5.44 | ND-50.1 | - | - |
| **Human** | 0.025 | 2.36 | 18.51 | BQL | ND | 1.69 | 11.83 |
| | 0.050 | 5.63 | 36.32 | 1.51 | 33.28 | 4.63 | 38.95 |
| | 0.100 | 10.62 | 133.47 | 3.44 | 125.14 | 2.85 | 34.02 |
| | 0.200 | 24.48 | 225.49 | 5.41 | 189.92 | 4.55 | 39.73 |
| | 0.400 | 68.13 | 526.37 | 10.44 | 444.76 | 23.03 | 202.99 |
| | 0.600 | 114.73 | 728.8 | 12.19 | 519.0 | 24.86 | 187.0 |

| | | | | | | | |
|---|---|---|---|---|---|---|---|
| Calculated based on mouse doses of 2 and 10 mg/kg, rabbit doses of 5 and 10 mg/kg ND Not Determined, BQL Below Quantitation Limit | | | | | | | |

## Claims

1. A compound: 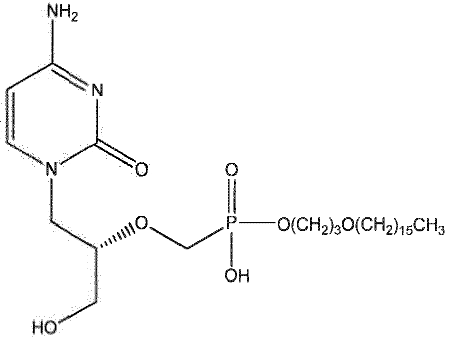 or a pharmaceutically acceptable salt thereof, for use in the therapeutic and/or prophylactic treatment of adenovirus in an immunodeficient subject, wherein said immunodeficient subject is a human.

2. A compound for use according to claim 1, wherein said immunodeficient subject experienced toxic side effects or nephrotoxicity from treatment with a compound selected from the group consisting of cidofovir, cyclic cidofovir, tenofovir, and adefovir.

3. A compound for use according to claim 1 or 2, wherein said immunodeficient subject has primary or acquired immunodeficiency.

4. A compound for use according to any of the preceding claims, wherein said immunodeficient subject has acquired immunodeficiency as a result of immunosuppressive therapy.

5. A compound for use according to any of the preceding claims, wherein said immunodeficient subject has acquired immunodeficiency as a result of cyclosporine treatment.

6. A compound for use according to any of the preceding claims, wherein said immunodeficient subject is a transplant patient.

7. A compound for use according to any of the preceding claims, wherein said immunodeficient subject is a renal transplant patient, a hepatic transplant patient, or a bone marrow transplant patient.

8. A compound for use according to any of the preceding claims, wherein said immunodeficient subject is suffering from chronic fatigue syndrome; and/or wherein the viral infection is resistant to treatment with a compound selected from the group consisting of cidofovir, cyclic cidofovir, tenofovir, and adefovir.

9. A compound for use according to claim 1, wherein said immunodeficient subject is infected with two or more viruses and said two or more viruses exhibit synergistic action.

11. A compound for use according to any of the preceding claims, wherein said immunodeficient subject is resistant to valganciclovir hydrochloride (or ganciclovir) or wherein said immunodeficient subject exhibits side effects to valganciclovir hydrochloride (or ganciclovir).

12. A compound for use according to any of the preceding claims, wherein said compound is administered to said immunodeficient subject at a dosage of 20 up to 5000 µg/kg.

13. A compound for use according to claim 1, wherein said compound is administered daily, every other day, once a week, or once every two weeks.

14. A compound for use according to any of the preceding claims, wherein said compound is administered to said immunodeficient subject at a dosage of less than 5 mg/kg.

## Patentansprüche

1. Verbindung: 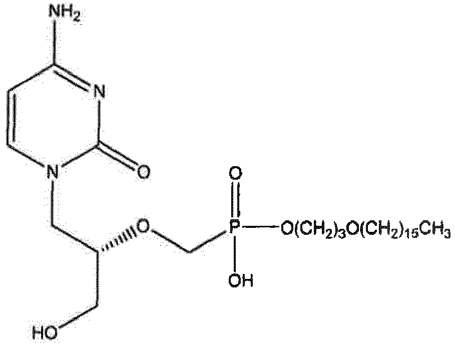 oder ein pharmazeutisch verträgliches Salz davon, zur Verwendung bei der therapeutischen und/oder prophylaktischen Behandlung eines Adenovirus in einem immundefizienten Probanden, wobei der immundefiziente Proband ein Mensch ist.

2. Verbindung zur Verwendung gemäß Anspruch 1, wobei bei dem immundefizienten Probanden toxische Nebenwirkungen oder Nephrotoxizität von einer Behandlung mit einer Verbindung auftraten, die ausgewählt ist aus der Gruppe bestehend aus Cidofovir, cyclischem Cidofovir, Tenofovir und Adefovir.

3. Verbindung zur Verwendung gemäß Anspruch 1 oder 2, wobei der immundefiziente Proband primäre oder erworbene Immundefizienz hat.

4. Verbindung zur Verwendung gemäß einem der vorangehenden Ansprüche, wobei der immundefiziente Proband erworbene Immundefizienz als Folge einer immunsuppressiven Therapie hat.

5. Verbindung zur Verwendung gemäß einem der vorangehenden Ansprüche, wobei der immundefiziente Proband erworbene Immundefizienz als Folge einer Cyclosporinbehandlung hat.

6. Verbindung zur Verwendung gemäß einem der vorangehenden Ansprüche, wobei der immundefiziente Proband ein Transplantationspatient ist.

7. Verbindung zur Verwendung gemäß einem der vorangehenden Ansprüche, wobei der immundefiziente Proband ein Nierentransplantationspatient, ein Lebertransplantationspatient oder ein Knochenmarktransplantationspatient ist.

8. Verbindung zur Verwendung gemäß einem der vorangehenden Ansprüche, wobei der immundefiziente Proband an chronischem Erschöpfungssyndrom leidet; und/oder wobei die Virusinfektion gegen eine Behandlung mit einer Verbindung resistent ist, die ausgewählt ist aus der Gruppe bestehend aus Cidofovir, cyclischem Cidofovir, Tenofovir und Adefovir.

9. Verbindung zur Verwendung gemäß Anspruch 1, wobei der immundefiziente Proband mit zwei oder mehr Viren infiziert ist und die zwei oder mehr Viren eine synergistische Wirkung aufweisen.

11. Verbindung zur Verwendung gemäß einem der vorangehenden Ansprüche, wobei der immundefiziente Proband gegen Valganciclovir-Hydrochlorid (oder Ganciclovir) resistent ist oder wobei der immundefiziente Proband Nebenwirkungen von Valganciclovir-Hydrochlorid (oder Ganciclovir) aufweist.

12. Verbindung zur Verwendung gemäß einem der vorangehenden Ansprüche, wobei die Verbindung dem immundefizienten Probanden in einer Dosierung von 20 bis zu 5000 µg/kg verabreicht wird.

13. Verbindung zur Verwendung gemäß Anspruch 1, wobei die Verbindung täglich, jeden zweiten Tag, einmal pro Woche oder einmal alle zwei Wochen verabreicht wird.

14. Verbindung zur Verwendung gemäß einem der vorangehenden Ansprüche, wobei die Verbindung dem immundefizienten Probanden in einer Dosierung von weniger als 5 mg/kg verabreicht wird.

## Revendications

1. Composé: 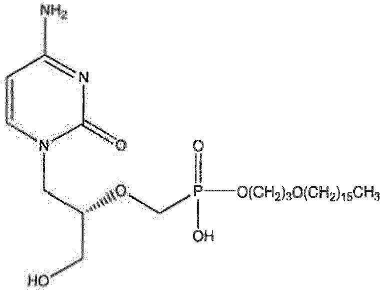 ou sel pharmaceutiquement acceptable de celui-ci, pouvant être utilisé dans le traitement thérapeutique et/ou prophylactique de l'adénovirus chez un sujet immunodéficient, dans lequel ledit sujet immunodéficient est un sujet humain.

2. Composé pour son utilisation selon la revendication 1, dans lequel ledit sujet immunodéficient a souffert des effets secondaires toxiques ou de la néphrotoxicité découlant d'un traitement avec un composé choisi dans le groupe constitué par le cidofovir, le cidofovir cyclique, le ténofovir, et l'adéfovir.

3. Composé pour son utilisation selon la revendication 1 ou 2, dans lequel ledit sujet immunodéficient souffre d'une immunodéficience primaire ou acquise.

4. Composé pour son utilisation selon l'une quelconque des revendications précédentes, dans lequel ledit sujet immunodéficient souffre d'une immunodéficience acquise résultant d'une thérapie immunosuppressive.

5. Composé pour son utilisation selon l'une quelconque des revendications précédentes, dans lequel ledit sujet immunodéficient souffre d'une immunodéficience acquise résultant d'un traitement à la cyclosporine.

6. Composé pour son utilisation selon l'une quelconque des revendications précédentes, dans lequel ledit sujet immunodéficient est un patient transplanté.

7. Composé pour son utilisation selon l'une quelconque des revendications précédentes, dans lequel ledit sujet immunodéficient est un patient transplanté rénal, un patient transplanté hépatique, ou un patient ayant subi une transplantation de moelle osseuse.

8. Composé pour son utilisation selon l'une quelconque des revendications précédentes, dans lequel ledit sujet immunodéficient souffre du syndrome de fatigue chronique ; et/ou chez lequel l'infection virale résiste au traitement avec un composé choisi dans le groupe constitué par le cidofovir, le cidofovir cyclique, le ténofovir, et l'adéfovir.

9. Composé pour son utilisation selon la revendication 1, dans lequel ledit sujet immunodéficient est infecté par deux virus ou plus et lesdits deux virus ou plus font preuve d'une action synergique.

11. Composé pour son utilisation selon l'une quelconque des revendications précédentes, dans lequel ledit sujet immunodéficient est résistant au chlorhydrate de valganciclovir (ou au ganciclovir) ou dans lequel ledit sujet immunodéficient souffre des effets secondaires du chlorhydrate de valganciclovir (ou du ganciclovir).

12. Composé pour son utilisation selon l'une quelconque des revendications précédentes, dans lequel ledit composé est administré audit sujet immunodéficient à une dose de 20 jusqu'à 5 000 µg/kg.

13. Composé pour son utilisation selon la revendication 1, dans lequel ledit composé est administré tous les jours, tous les 2 jours, une fois par semaine, ou une fois toutes les 2 semaines.

14. Composé pour son utilisation selon l'une quelconque des revendications précédentes, dans lequel ledit composé est administré audit sujet immunodéficient à une dose inférieure à 5 mg/kg.
